# EUROPEAN PATENT SPECIFICATION

(11) **EP 2 496 092 B1**
(45) Date of publication and mention of the grant of the patent: **28.03.2018**
(21) Application number: 10778936.4
(22) Date of filing: 02.11.2010
(51) Int. Cl.: A23L 33/115

(54) **COMPOSITION COMPRISING CELLS AND A POLYUNSATURATED FATTY ACID HAVING AT LEAST 20 CARBON ATOMS (LC-PUFA)**
ZUSAMMENSETZUNG MIT ZELLEN UND EINER MEHRFACH UNGESÄTTIGTEN FETTSÄURE MIT MINDESTENS 20 KOHLENSTOFFATOMEN (LC-PUFA)
COMPOSITION CONTENANT DES CELLULES ET UN ACIDE GRAS POLYINSATURÉ COMPORTANT AU MOINS 20 ATOMES DE CARBONE (LC-PUFA)

(30) Priority: 03.11.2009 US 257772 P; 22.11.2009 WO PCT/EP2009/065593; 22.11.2009 WO PCT/EP2009/065592
(43) Date of publication of application: 12.09.2012
(73) Proprietor: DSM IP Assets B.V., 6411 TE Heerlen (NL)
(72) Inventor: VERKOEIJEN, Daniel, Stewartsville, NJ 08886 (US); BIJL, Hendrik, Louis, NL-3131 ZD Vlaardingen (NL); ZUUR, Kristian, NL-2645 HK Delfgauw (NL)
(74) Representative: Hoyng Rokh Monegier LLP
(86) International application number: PCT/EP2010/066598
(87) International publication number: WO 2011/054800

(56) References cited:
- WO-A2-97/37032
- US-A- 5 882 703
- US-A1- 2004 049 062
- US-A1- 2006 122 410
- NISHA, A., MUTHUKUMAR, S.P., AND VENKATESWARAN, G.: "Safety evaluation of arachidonic acid rich Mortierella alpina biomass in albino rats-A subchronic study", REGULATORY TOXICOLOGY AND PHARMACOLOGY, vol. 53, 19 January 2009 (2009-01-19), pages 186-194, XP002599293, ACADEMIC PRESS,NEW YORK, NY, ISSN: 0273-2300

## Description

The present invention relates to a composition comprising cells and a polyunsaturated fatty acid having at least 20 carbon atoms (LC-PUFA), to a process for drying a composition comprising cells and a LC-PUFA, and to a process for obtaining an LC-PUFA or an oil containing an LC-PUFA from a composition comprising cells and an LC-PUFA.

The invention is defined by the claims. LC-PUFAs can be produced by micro-organisms in a fermentation process. LC-PUFAs can also be produced in plants. The microorganisms or plant parts containing the LC-PUFA can then be pre-treated after which LC-FUFA or oil containing the LC-PUFA can be isolated.

For instance, WO 2006/085672, describes a process wherein an LC-PUFA is isolated from a microbial biomass. Wet cells are dried in a two-stage drying process. Drying temperatures of 120 ºC and higher are used, and dried cells having a moisture content of 1-2 wt.% are obtained.

US 2004/049062 describes a microbial polyunsaturated fatty acid (PUFA)-containing oil with a high triglyceride content and a high oxidative stability. Further, a method is described for the recovery of such oil from a microbial biomass derived from a pasteurised fermentation broth, wherein the microbial biomass is subjected to extrusion to form granular particles, dried and the oil then extracted from the dried granules using an appropriate solvent.

US 2006/122410 describes a process for preparing a biomass, such as from a microbial fermentation, for an extraction process to separate desired chemicals, nutritional products, bioactive components, proteins, carbohydrates, and lipids, from the biomass. The method comprises freezing the biomass at or below its brittleness temperature, comminuting the frozen biomass at a temperature no higher than the brittleness temperature to produce a comminuted biomass; and agglomerating the comminuted biomass to produce an agglomerated biomass.

Nisha et al ("Safety evaluation of arachidonic acid rich Mortierella alpina biomass in albinorats - A subchronic study", Reg. Tox. and Pharm., vol. 53, 2009-01-19, pp. 186-194) concerns a safety evaluation of arachidonic acid rich *Mortierella alpina* biomass which was carried out in Wistar rats by acute and subchronic oral toxicity studies. Biomass was prepared by harvesting the biomass by suction filtration and freeze drying. This was subsequently powdered and used for experimental diet preparation.

US 5882703 describes a process for producing arachidonic acid. In one embodiment, Mortierella sect. *schmuckeri* microorganisms are cultured in fermentation medium, preferably containing a component of a complex nitrogen source. Further disclosed is a food product which includes Mortierella sect. *schmuckeri* microorganisms or lipid isolated from such microorganisms to enhance the arachidonic acid content of the food product.

It is possible to isolate the LC-PUFA and/or oil containing the LC-PUFA from the LC-PUFA-containing composition immediately after its production. However, in practice the LC-PUFA-containing composition is often stored and/or transported before further use such as isolation of the LC-PUFA and/or oil containing the LC-PUFA.

It is now found that LC-PUFA-containing compositions are susceptible to self-heating. *Viz.* during storage, the temperature can increase spontaneously, ultimately resulting in unexpected explosions and fires. It is further found that this susceptibility increases with increasing LC-PUFA content, and with increasing number of double bonds of the LC-PUFAs.

It is an object of the invention to provide a composition comprising (i) LC-PUFA and (ii) cells, which composition is safer.

The invention now provides a composition comprising (i) a polyunsaturated fatty acid having at least 20 carbon atoms (LC-PUFA) and (ii) cells, wherein the composition has a moisture content between 1 and 20 wt.%, and an oil content of at least 10 wt.%, wherein the oil contains at least 40 wt.% of PUFAs with at least three double bonds with respect to the total fatty acids in the oil, which composition has a thermal induction time (T.I.T.) of > 24 hours at 40 ºC.

The composition according to the invention has the advantage that its safety is improved, and that the risk of spontaneous temperature increase, unexpected explosions and fires is decreased. A further advantage of the composition of the invention is that storage of the composition does not negatively affect the quality of the LC-PUFA or oil containing the LC-PUFA, or at least imparts the quality to a lesser extent.

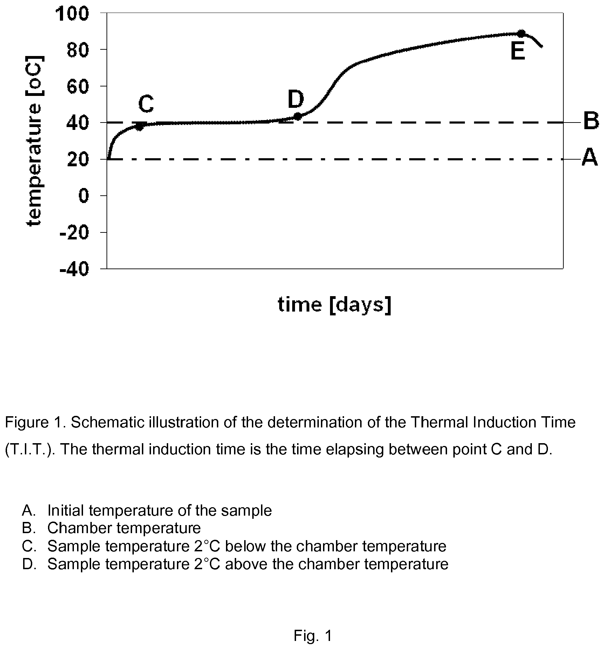

The composition according to the invention has a thermal induction time (T.I.T.) of > 24 hours at 40 ºC. The T.I.T. of the composition is determined using a heat accumulation storage test as described in Recommendations on the Transport of Dangerous Goods, Manual of Tests and Criteria, Section 28.4.4, Test H.4, United Nations, New York, 1999, with the following adaptations and specifications: A glass 0.5 I Dewar vessel with an internal diameter of 57 mm and a height of 210 mm is used. The heat loss of the Dewar vessel is 16 mW/K. The sample size is 75% of the volume of the Dewar vessel. The Dewar vessel is closed with a rubber stopper with a height of approximately 50 mm, loosely tightened to allow for respiration. A thermocouple is inserted in the Dewar vessel through a hole in the centre of the stopper. The Dewar vessel containing the sample, which have an initial temperature of 20 °C, are placed in a chamber that is controlled at 40 °C, and the temperature of the sample is monitored using the thermocouple. The thermal induction time is defined as time elapsing between moment at which the temperature of the sample reaches the temperature of 2°C below the chamber temperature (hence 38 °C), and the moment at which the temperature of the sample reaches the temperature of 2°C above the chamber temperature (hence 42 °C). The determination of the T.I.T. is illustrated in figure 1.

Preferably the composition according to the invention has a T.I.T. of at least 2 days (48 hours), preferably at least 3 days (72 hours), preferably at least 4 days (96 hours), preferably at least 5 days, measured at 40 °C. The T.I.T. may be at least 8 days, for instance at least 10 days, measured at 40 °C. There is no specific upper limit for the T.I.T.. The T.I.T. may be less than 25 days, for instance less than 20 days, measured at 40 °C.

The composition having the increased T.I.T. according to the invention can be obtained based on the teaching provided by the invention.

The composition may be a dried composition. It is found that the T.I.T. increases if the drying temperature is decreased. It is further found that it is advantageous to decrease the drying temperature if the content of LC-PUFAs in the composition or the number of double bonds of the LC-PUFAs is higher.

For instance, the drying temperature may be below 40 ºC, preferably below 35 ºC, more preferably below 33 º, more preferably below 30 ºC, more preferably below 25 ºC. As used herein, the drying temperatures refer to the temperature of the product in the dryer. For instance, if the drier is a fluid bed dryer, the drying temperature refers to the temperature of the bed.

Accordingly, the invention also provides a process for providing a composition according to the invention, wherein the process comprises drying a composition comprising cells and a LC-PUFA, the process comprising drying the composition at a temperature of below 40 ºC, preferably below 35 ºC, preferably below 33 ºC, preferably below 30 ºC, preferably below 25 ºC.

The drying may be by any suitable method. Drying may be performed in any suitable dryer. Preferably a dryer is used which prevents or minimizes the formation of hot spots. In a preferred embodiment drying is effected using a fluid bed dryer.

It is found that the T.I.T. increases with decreasing drying time.

In a preferred embodiment, drying is effected using conditioned air. Preferably air is used having a dew point of < 15 ºC, preferably < 10 ºC, preferably < 5 ºC. Decreasing the dew point has the advantage that preferred moisture contents can efficiently be achieved at preferred (low) drying temperatures.

It is further found that the T.I.T. increases with increases if the moisture content of the composition increases. The (e.g. dried) composition has a moisture content between 1 and 20 wt.%, preferably at least 2 wt.%, preferably at least 3 wt.%, preferably at least 4 wt.%, for instance below 15 wt.%, for instance below 12 wt.%, for instance below 10 wt.%, for instance below 9 wt.%. Decreasing the moisture content below the preferred values is found to increase the microbial stability of the composition.

As used herein, the moisture content is calculated on a wet weight basis, i.e. on the basis of the total weight of the composition (including dry matter, oil, and moisture). It and can be determined by the skilled person, for instance by evaporating the water at a temperature of 105 °C, and determining the weight of the evaporated moisture.

It is further found that avoiding the formation of free radicals during processing of the composition can result increased values for the T.I.T.. Based on this insight, the skilled person can avoid steps resulting in the formation of free radicals. Accordingly, it is generally preferable to minimize exposure of the cells to circumstances that can promote the formation of free radicals, e.g. exposure to high temperatures and/or to oxygen.

If the cells are microbial cells, advantageously a fermentation broth containing the cells is heated such as to sufficiently kill off the enzymes that may be present in the fermentation broth. Preferred heating protocols are described in WO 97/037032 and WO 2004/001021. Preferably a fermentation broth having a low dissolved oxygen content, for instance < 10 ppm, for instance < 5 ppm, for instance < 2 ppm, for instance < 1 ppm, is heated. Killing off the enzymes, in particular using the protocols as referred to hereinabove, may result in increased values of the T.I.T..

The composition according to the invention has an oil content of at least 10 wt.%, for instance at least 20 wt.%, for instance at least 30 wt.%, for instance at least 40 wt.%. The oil content may be below 70 wt.%, for instance below 60 wt.%. The oil content may be determined by methods known to the skilled person. A suitable method for determining the oil content of the composition as used herein is by using a Soxhlet extraction using n-hexane as the solvent, wherein the composition subjected to the extraction has a moisture content < 15 wt.% and wherein the composition and cells are comminuted (to ensure that all oil is released from the cells and can dissolve into the solvent). As used herein, the oil content is calculated on a dry basis, i.e. on the basis of the total dry weight of the composition (including dry matter and oil, but excluding moisture).

The composition according to the invention has an oil content as defined in the claims or as defined in the preferred embodiments above, wherein the composition of the oil is as defined in the claims or as defined in the preferred embodiments described below.

The composition comprises an oil which comprises at least 40 wt.% of PUFAs with at least 3 double bonds with respect to the total fatty acids in the oil, for instance below 80 wt.%, for instance below 70 wt.%, for instance below 60 wt.% of PUFAs with at least 3 double bonds with respect to the total fatty acids in the oil. As used herein, the wt.% of PUFAs with at least 3 double bonds refers to the sum of all PUFAs with at least 3 double bonds.

In a preferred embodiment, the composition comprises an oil which comprises at least 10 wt.%, for instance at least 20 wt.%, for instance at least 30 wt.%, for instance at least 40 wt.% of arachidonic acid (ARA) with respect to the total fatty acids in the oil, for instance below 80 wt.%, for instance below 70 wt.%, for instance below 60 wt.% ARA with respect to the total fatty acids in the oil.

In a preferred embodiment, the composition comprises an oil which comprises at least 10 wt.%, for instance at least 20 wt.%, for instance at least 30 wt.%, for instance at least 40 wt.% of docosahexaenoic acid (DHA) with respect to the total fatty acids in the oil, for instance below 80 wt.%, for instance below 70 wt.%, for instance below 60 wt.% DHA with respect to the total fatty acids in the oil.

A suitable method for determining the composition of the oil as used herein is to extract the oil from the composition using the Soxhlet extraction using n-hexane as described hereinabove, and to determine the fatty acid composition of the extracted oil.

It is preferred to select lower drying temperatures and shorter residence times in the dryer if the oil content and/or number of double bonds is relatively high.

Based on the teaching herein it is possible to high values for the T.I.T. even for compositions with a high oil content and/or for compositions containing an oil with a high concentration of PUFAs with at least 3 double bonds.

As used herein, the following abbreviations are used throughout the entire application:
PUFA refers to a polyunsaturated fatty acid
LC-PUFA (long chain polyunsaturated fatty acid) refers to a PUFA having at least 20 carbon atoms
HUFA (highly unsaturated fatty acid) refers to a PUFA having at least three double bonds
LC-HUFA (long chain highly unsaturated fatty acid) refers to a polyunsaturated fatty acid having at least 20 carbon atoms and at least three double bonds.

The invention is not limited to a specific LC-PUFA. In an embodiment of the invention, the LC-PUFA has at least three double bonds. In a further embodiment of the invention, the LC-PUFA has at least four double bonds. The benefits of the invention are even more pronounced for LC-PUFAs having an increasing number of double bonds, as the susceptibility to self heating increases with increasing number of double bonds.

The LC-PUFA may be an ω-3 LC-PUFA or an ω-6 LC-PUFA
LC-PUFAs include for instance:
dihomo-γ-linolenic acid (DGLA, 20:3 ω-6)
arachidonic acid (ARA, 20:4 ω-6)
eicosapentaenoic acid (EPA, 20:5 ω-3)
docosapentaenoic acid (DPA, 22:5 ω-3, or DPA 22:5, ω-6),
docosahexaenoic acid (DHA: 22:6 ω-3)

Preferred LC-PUFAs include arachidonic acid (ARA) and docosahexaenoic acid (DHA). In particular ARA is preferred.

The composition according to the invention comprises cells. The cells may be any cells containing and/or having produced the LC-PUFA.

In an embodiment of the invention, the cells are microbial cells (microorganisms).

Examples of microbial cells are yeast cells, bacterial cells, fungal cells, and algal cells. Fungi are preferred, preferably of the order *Mucorales.* Example are *Mortierella, Phycomyces, Blakeslea, Aspergillus, Thraustochytrium, Pythium or Entomophthora.* The preferred source of arachidonic acid (ARA) is from *Mortierella alpina.* Algae can be dinoflagellate and/or include *Porphyridium, Nitszchia, or Crypthecodinium (*e.g. *Crypthecodinium cohnii).* Yeasts include those of the genus *Pichia or Saccharomyces, such as Pichia ciferii.* Bacteria can be of the genus *Propionibacterium.*

In an embodiment of the invention, the composition comprises a fungus of the genus *Mortierella,* preferably of the species *Mortierella alpina,* wherein preferably the LC-PUFA is ARA or DGLA.

In an embodiment of the invention the composition comprises a fungus of the order *Thraustochytriales,* for instance from the genus *Thraustochytrium* or *Schizochytrium*, and wherein preferably the LC-PUFA is DHA and/or EPA.

In an embodiment of the invention, the composition comprises an algae of the genus *Crypthecodinium,* preferably of the species *Crypthecodinium cohnii*, wherein preferably the LC-PUFA is DHA.

Preferably, the composition is a (dried) composition obtained or obtainable by a drying process disclosed herein.

The composition may be a microbial biomass comprising a microorganism and a LC-PUFA. Preferred microorganisms and LC-PUFAs are mentioned hereinabove.

In a possible embodiment of the invention a composition comprising microorganisms (microbial cells) according to the invention is obtained in a process comprising heating (also referred to as pasteurization or sterilization) a fermentation broth comprising the microbial cells, dewatering the microbial cells, e.g. by filtration, and drying the microbial cells in a process described hereinabove. In a preferred embodiment, the dewatered microbial cells are granulated prior to drying, preferably by extrusion. Preferably granulation, e.g. extrustion is performed at a temperature below 25 ºC. A preferred process is described in WO 97/037032.

The composition may be stored for any suitable period. The composition may for instance be stored for at least 1 day, for instance at least 1 week, for instance at least 2 weeks, for instance at least 1 months, for instance at least 3 months. There is no specific upper limit for the storage period. The composition, may for instance be stored for less than 12 months, for instance less than 6 months.

The invention further comprises a process for obtaining a LC-PUFA or an oil comprising a LC-PUFA, comprising isolating said LC-PUFA or oil comprising a LC-PUFA from a composition according the invention or from a composition obtained or obtainable by the process according to the invention.

The lipid LC-PUFA or oil comprising the LC-PUFA may be obtained by extraction the LC-PUFA or oil comprising the LC-PUFA from the composition, preferably by solvent extraction. Any suitable solvent may be used, for instance a C₁₋₁₀ alkyl ester (e.g. ethyl or butyl acetate), toluene, a C₁₋₃ alcohol (e.g. methanol, propanol), a C₃₋₆ alkanes (e.g. hexane) or a supercritical fluid (e.g. liquid CO₂ or supercritical propane). Preferably, the solvent is a non-polar solvent, for instance a C₃-C₈ alkane (preferably hexane) or a supercritical fluid (preferably supercritical CO₂ or supercritical propane). Preferred extraction procedures are described in WO 97/037032.

Purifying of the oil may comprise degumming, refining, bleaching and/or deodorizing. These are known steps, and can be carried out by the skilled person. In a preferred embodiment, deodorizing is effected at a temperature below 200 ºC, preferably below 190 ºC, preferably below 185 ºC. Decreasing the deodorization temperature to below the preferred values improves the quality of the oil.

The invention further provides a process for obtaining a food product, in particular an infant formula, comprising obtaining a LC-PUFA or an oil comprising a LC-PUFA from the composition according to the invention, and incorporating said LC-PUFA or oil comprising said LC-PUFA in said food product.

Further preferred aspects, embodiments and features are disclosed in the claims

Preferred features and characteristics of one embodiment and/or aspect of the invention are applicable to another embodiment *mutatis mutandis.* As used herein, the preferred features and characteristics of the LC-PUFA apply to the LC-PUFAs in all aspects and embodiments of the invention.

The invention is further disclosed with reference to the following examples.

### EXAMPLES

### Example 1

Fermentation broth of *Mortierella alpina,* obtained after 8 days of fermentation was pasteurized at 70 ºC for 1 hour. The pasteurized broth was filtered, resulting in a filter cake have a moisture content of 50 wt.%. The filter cake was crumbled and extruded at a temperature below 15 ºC. The extrudate (diameter 3 mm) was dried in a continuous fluid bed drier with three zones to a moisture content of 7%. In the first zone the bed temperature was 32 °C and the air temperature 50 ºC (T_{dew point} = 15°C).
1^{st} zone: bed temperature 32 °C, the air temperature 50 ºC (T_{dew point} = 15°C): 45 minutes
2^{nd} zone: bed temperature 32 ºC, air temperature 35 ºC (T_{dew point} = 1°C): 45 minutes
3^{rd} zone: bed temperature 15 ºC, air temperature 15 ºC (T_{dew point} = 1°C): 30 minutes
The oil content of the dried biomass was 39%. The ARA content was 46% with respect to the total fatty acids in the oil.
Thermal induction time (T.I.T.) measured at 40 ºC : 9 days.

### COMPARATIVE EXPERIMENT A

The fermentation and pasteurization is repeated. The wet cells are recovered using a continuous dehydrator and disrupted, and then drying is carried out by hot air drying (hot air temperature 120 ºC) with a vibrating fluidized bed dried to a moisture content of 1 wt.%. The dried cells are cooled by supplying room temperature air in the fluidized bed. ARA and oil content are as in example 1.
Thermal induction time (T.I.T.) measured at 40 ºC : < 12 hours

### DESCRIPTION OF THE FIGURE

Figure 1 shows a Schematic illustration of the determination of the Thermal Induction Time (T.I.T.).

## Claims

1. Composition comprising (i) a polyunsaturated fatty acid having at least 20 carbon atoms (LC-PUFA) and (ii) cells, wherein the composition has a moisture content between 1 and 20 wt%, and an oil content of at least 10 wt%, wherein the oil contains at least 40 wt% of PUFAs with at least three double bonds with respect to the total fatty acids in the oil, which composition has a thermal induction time (T.I.T.) of > 24 hours at 40 ºC.

2. Composition according to claim 1, which has a moisture content of between 2 and 15 wt.%, preferably between 3 and 12 wt.%, preferably between 3.5 and 10 wt.%, preferably between 4 and 9 wt.%.

3. Composition according to claim 1 or 2, which has an oil content of at least 20 wt.%, for instance at least 30 wt.%, for instance at least 40 wt.%, for instance below 70 wt.%, for instance below 60 wt.%.

4. Composition according to any preceding claim, wherein the composition comprises an oil which comprises below 80 wt.%, for instance below 70 wt.%, for instance below 60 wt.% of PUFAs with at least 3 double bonds with respect to the total fatty acids in the oil.

5. Composition according to any preceding claim, wherein the LC-PUFA is an ω-3 or an ω-6 PUFA.

6. Composition according to any preceding claim, wherein the LC-PUFA is selected from dihomo-γ-linolenic acid (DGLA, 20:3 ω-6), arachidonic acid (ARA, 20:4 ω-6), eicosapentaenoic acid (EPA, 20:5 ω-3), docosahexaenoic acid (DHA: 22:6 ω-3), docosapentaenoic acid (DPA 22:5 ω-3, or DPA 22:5, ω-6).

7. Composition according to any preceding claim, wherein the LC-PUFA is ARA or DHA.

8. Composition according to any preceding claim, wherein the cells are microbial cells.

9. Composition according to claim 8, wherein the microbial cells are yeast cells, bacterial cells, fungal cells, or algal cells.

10. Composition according to any preceding claim, wherein the composition comprises a microorganism of the genus *Mortierella,* preferably of the species *Mortierella alpina,* and wherein preferably the LC-PUFA is ARA.

11. Composition according to any one of claims 1 to 9, wherein the composition comprises a microorganism of the order Thraustochytriales, for instance of the genus *Thraustochytrium or Schizochytrium*, and wherein preferably the LC-PUFA is DHA or EPA.

12. Composition according to any one of claims 1 to 9, wherein the composition comprises a microorganism of the genus *Crypthecodinium,* preferably of the species *Crypthecodinium cohnii*, and wherein preferably the LC-PUFA is DHA.

13. Composition according to any preceding claim, wherein the composition is obtainable by the process according to any one of claims 14-16.

14. Process for providing a composition according to any one of claims 1-13, wherein the process comprises drying a composition comprising cells and a LC-PUFA, the process comprising drying the composition at a temperature of below 40 ºC, preferably below 35 ºC, preferably below 33 ºC, preferably below 30 ºC, preferably below 25 ºC.

15. Process according to claim 14, wherein the process comprises contacting the composition with conditioned air which preferably has a dew point of ≤ 15 ºC, preferably < 10 ºC, preferably < 5 ºC.

16. Process according to claim 14 or 15, comprising drying the composition using a fluid bed dryer.

17. Process for obtaining a LC-PUFA or an oil comprising a LC-PUFA, the process comprising isolating said LC-PUFA or oil comprising a LC-PUFA from a composition according to any of claims 1-13.

18. Process for obtaining a food product, in particular an infant formula, said process comprising obtaining a LC-PUFA or an oil comprising a LC-PUFA according to the process of claim 17, and incorporating said LC-PUFA or oil comprising said LC-PUFA in said food product.

## Patentansprüche

1. Zusammensetzung umfassend (i) eine mehrfach ungesättigte Fettsäure mit mindestens 20 Kohlenstoffatomen (LC-PUFA) und (ii) Zellen, wobei die Zusammensetzung einen Feuchtigkeitsgehalt von zwischen 1 und 20 Gew.-% und einen Ölgehalt von mindestens 10 Gew.-% aufweist, wobei das Öl mindestens 40 Gew.-% PUFAs mit mindestens drei Doppelbindungen in Bezug auf die Gesamtfettsäuren in dem Öl enthält, und wobei die Zusammensetzung eine thermische Induktionszeit (Thermal Induction Time, TIT) von > 24 Stunden bei 40°C aufweist.

2. Zusammensetzung nach Anspruch 1, die einen Feuchtigkeitsgehalt von zwischen 2 und 15 Gew.-%, vorzugsweise zwischen 3 und 12 Gew.-%, vorzugsweise zwischen 3,5 und 10 Gew.-%, vorzugsweise zwischen 4 und 9 Gew.-%, aufweist.

3. Zusammensetzung nach Anspruch 1 oder 2, die einen Ölgehalt von mindestens 20 Gew.-%, zum Beispiel mindestens 30 Gew.-%, zum Beispiel mindestens 40 Gew.-%, zum Beispiel unter 70 Gew.-%, zum Beispiel unter 60 Gew.-%, aufweist.

4. Zusammensetzung nach einem vorhergehenden Anspruch, wobei die Zusammensetzung ein Öl umfasst, das unter 80 Gew.-%, zum Beispiel unter 70 Gew.-%, zum Beispiel unter 60 Gew.-% PUFAs mit mindestens 3 Doppelbindungen in Bezug auf die Gesamtfettsäuren in dem Öl umfasst.

5. Zusammensetzung nach einem vorhergehenden Anspruch, wobei es sich bei der LC-PUFA um eine ω-3 oder ω-6-PUFA handelt.

6. Zusammensetzung nach einem vorhergehenden Anspruch, wobei die LC-PUFA aus Dihomo-γ-Linolensäure (DGLA, 20:3 ω-6), Arachidonsäure (ARA, 20:4 ω-6), Eicosapentaensäure (EPA, 20:5 ω-3), Docosahexaensäure (DHA, 22:6 ω-3), Docosapentaensäure (DPA, 22:5 ω-3 oder DPA, 22:5 ω-6) ausgewählt ist.

7. Zusammensetzung nach einem vorhergehenden Anspruch, wobei es sich bei der LC-PUFA um ARA oder DHA handelt.

8. Zusammensetzung nach einem vorhergehenden Anspruch, wobei es sich bei den Zellen um Mikroorganismenzellen handelt.

9. Zusammensetzung nach Anspruch 8, wobei es sich bei den Mikroorganismenzellen um Hefezellen, Bakterienzellen, Pilzzellen oder Algenzellen handelt.

10. Zusammensetzung nach einem vorhergehenden Anspruch, wobei die Zusammensetzung einen Mikroorganismus der Gattung *Mortierella*, vorzugsweise der Art *Mortierella alpina*, umfasst und wobei es sich vorzugsweise bei der LC-PUFA um ARA handelt.

11. Zusammensetzung nach einem der Ansprüche 1 bis 9, wobei die Zusammensetzung einen Mikroorganismus der Ordnung *Thraustochytriales*, zum Beispiel der Gattung *Thraustochytrium* oder *Schizochytrium*, umfasst und wobei es sich vorzugsweise bei der LC-PUFA um DHA oder EPA handelt.

12. Zusammensetzung nach einem der Ansprüche 1 bis 9, wobei die Zusammensetzung einen Mikroorganismus der Gattung *Crypthecodinium*, vorzugsweise der Art *Crypthecodinium cohnii*, umfasst und wobei es sich vorzugsweise bei der LC-PUFA um DHA handelt.

13. Zusammensetzung nach einem vorhergehenden Anspruch, wobei die Zusammensetzung durch das Verfahren nach einem der Ansprüche 14-16 erhältlich ist.

14. Verfahren zum Bereitstellen einer Zusammensetzung nach einem der Ansprüche 1-13, wobei das Verfahren das Trocknen einer Zusammensetzung, die Zellen und eine LC-PUFA umfasst, wobei das Verfahren das Trocknen der Zusammensetzung bei einer Temperatur von unter 40°C, vorzugsweise unter 35°C, vorzugsweise unter 33°C, vorzugsweise unter 30°C, vorzugsweise unter 25°C, umfasst.

15. Verfahren nach Anspruch 14, wobei das Verfahren das Inkontaktbringen der Zusammensetzung mit aufbereiteter Luft, die vorzugsweise einen Taupunkt von ≤ 15°C, vorzugsweise < 10°C, vorzugsweise < 5°C aufweist, umfasst.

16. Verfahren nach Anspruch 14 oder 15, bei dem man die Zusammensetzung unter Einsatz eines Wirbelbetttrockners trocknet.

17. Verfahren zum Gewinnen einer LC-PUFA oder eines Öls, das eine LC-PUFA umfasst, bei dem man die LC-PUFA oder das Öl, das eine LC-PUFA umfasst, aus einer Zusammensetzung nach einem der Ansprüche 1-13 isoliert.

18. Verfahren zum Gewinnen eines Nahrungsmittelprodukts, insbesondere einer Babynahrung, bei dem man eine LC-PUFA oder ein Öl, das eine LC-PUFA umfasst, gemäß dem Verfahren nach Anspruch 17 gewinnt und die LC-PUFA oder das Öl, das die LC-PUFA umfasst, in das Nahrungsmittelprodukt einarbeitet.

## Revendications

1. Composition comprenant (i) un acide gras polyinsaturé ayant au moins 20 atomes de carbone (AGPI-LC) et (ii) des cellules, où la composition possède une teneur en humidité comprise entre 1 et 20% en poids, et une teneur en huile d'au moins 10% en poids, où l'huile contient au moins 40% en poids d'AGPI ayant au moins trois doubles liaisons par rapport au acides gras totaux dans l'huile, laquelle composition possédant un temps d'induction thermique (T.I.T.) > 24 heures à 40°C.

2. Composition selon la revendication 1, possédant une teneur en humidité comprise entre 2 et 15% en poids, préférablement entre 3 et 12% en poids, préférablement entre 3,5 et 10% en poids, préférablement entre 4 et 9% en poids.

3. Composition selon la revendication 1 ou 2, possédant une teneur en huile d'au moins 20% en poids, par exemple d'au moins 30% en poids, par exemple d'au moins 40% en poids, par exemple inférieure à 70% en poids, par exemple inférieure à 60% en poids.

4. Composition selon l'une quelconque des revendications précédentes, où la composition comprend une huile qui comprend moins de 80% en poids, par exemple moins de 70% en poids, par exemple moins de 60% en poids, d'AGPI ayant au moins 3 doubles liaisons par rapport au acides gras totaux de l'huile.

5. Composition selon l'une quelconque des revendications précédentes, où l'AGPI-LC est un AGPI ω-3 ou ω-6.

6. Composition selon l'une quelconque des revendications précédentes, où l'AGPI-LC est choisi parmi l'acide dihomo-γ-linolénique (DGLA, 20:3 ω-6), l'acide arachidonique (ARA, 20:4 ω-6), l'acide eicosapentaénoïque (EPA, 20:5 ω-3), l'acide docosahexaénoïque (DHA, 22:6 ω-3), l'acide docosapentaénoïque (DPA, 22:5 ω-3 ou DPA, 22:5 ω-6).

7. Composition selon l'une quelconque des revendications précédentes, où l'AGPI-LC est l'ARA ou le DHA.

8. Composition selon l'une quelconque des revendications précédentes, où les cellules sont des cellules microbiennes.

9. Composition selon la revendication 8, où les cellules microbiennes sont des cellules de levure, des cellules bactériennes, des cellules fongiques, ou des cellules algales.

10. Composition selon l'une quelconque des revendications précédentes, où la composition comprend un microorganisme du genre *Mortierella*, préférablement de l'espèce *Mortierella alpina*, et où préférablement l'AGPI-LC est l'ARA.

11. Composition selon l'une quelconque des revendications 1 à 9, où la composition comprend un microorganisme de l'ordre *Thraustochytriales*, par exemple du genre *Thraustochytrium* ou *Schizochytrium*, et où préférablement l'AGPI-LC est le DHA ou l'EPA.

12. Composition selon l'une quelconque des revendications 1 à 9, où la composition comprend un microorganisme du genre *Crypthecodinium*, préférablement de l'espèce *Crypthecodinium cohnii*, et où préférablement l'AGPI-LC est le DHA.

13. Composition selon l'une quelconque des revendications précédentes, où la composition peut être obtenue par le procédé selon l'une quelconque des revendications 14-16.

14. Procédé de fourniture d'une composition selon l'une quelconque des revendications 1-13, où le procédé comprend le séchage d'une composition comprenant des cellules et un AGPI-LC, le procédé comprenant le séchage de la composition à une température inférieure à 40°C, préférablement inférieure à 35°C, préférablement inférieure à 33°C, préférablement inférieure à 30°C, préférablement inférieure à 25°C.

15. Procédé selon la revendication 14, où le procédé comprend la mise en contact de la composition avec un air conditionné ayant de préférence un point de rosée ≤ 15°C, préférablement < 10°C, préférablement < 5°C.

16. Procédé selon la revendication 14 ou 15, comprenant le séchage de la composition à l'aide d'un séchoir à lit fluidisé.

17. Procédé d'obtention d'un AGPI-LC ou d'une huile comprenant un AGPI-LC, le procédé comprenant l'isolement dudit AGPI-LC ou de ladite huile comprenant un AGPI-LC à partir d'une composition selon l'une quelconque des revendications 1-13.

18. Procédé d'obtention d'un produit alimentaire, en particulier d'une préparation pour nourrissons, ledit procédé comprenant l'obtention d'un AGPI-LC ou d'une huile comprenant un AGPI-LC selon le procédé selon la revendication 17, et l'incorporation dudit AGPI-LC ou de l'huile comprenant ledit AGPI-LC dans ledit produit alimentaire.
